# FASCICULE DE BREVET EUROPEEN

(11) **EP 0 613 372 B1**
(45) Date de publication et mention de la délivrance du brevet: **27.12.2000**
(21) Numéro de dépôt: 93920896.3
(22) Date de dépôt: 17.09.1993
(51) Int. Cl.: A61K 31/57

(54) **IMPLANTS SOUS-CUTANES A BASE DE DERIVES DE NOMEGESTROL**
SUBKUTANE IMPLANTATE BESTEHEND AUS NOMGESTROLDERIVATEN
SUBCUTANEOUS IMPLANTS BASED ON NOMEGESTROL DERIVATIVES

(30) Priorité: 21.09.1992 FR 9211422
(43) Date de publication de la demande: 07.09.1994
(73) Titulaire: LABORATOIRE THERAMEX S.A., 98000 Monaco (MC)
(72) Inventeur: LANQUETIN, Michel Quartier l'Adrech, F-06340 La Trinité (FR); THOMAS, Jean Louis, F-94220 Charenton-le-Pont (FR); PARIS, Jacques Le Clos de Cimiez, F-06110 Nice (FR); COUTINHO, Elsimar, Rua do Limoeiro no.1 Salvador, Bahia (BR)
(74) Mandataire: Burtin, Jean-François
(86) Numéro de dépôt international: FR9300900
(87) Numéro de publication internationale: WO9406437

(56) Documents cités:
- WO-A-92/01706
- FR-A- 2 271 833
- CONTRACEPTION vol. 47, no. 1 , Janvier 1993 pages 97 - 105 COUTINHO, E.M. 'ONE YEAR CONTRACEPTION WITH A SINGLE SUBDERMAL IMPLANT CONTAINING NOMEGESTEROL ACETATE (UNIPLANT)'
- JOURNAL OF PHARMACEUTICAL SCIENCES vol. 65, no. 4 , Avril 1976 pages 488 - 492 CHIEN, Y.W. ET AL 'CONTROLLED DRUG RELEASE FROM POLYMERIC DELIVERY DEVICES IV: IN VITRO-IN VIVO CORRELATION OF SUBCUTANEOUS RELEASE OF NORGESTOMET FROM HYDROPHILIC IMPLANTS' cité dans la demande
- JOURNAL OF PHARMACEUTICAL SCIENCE vol. 68, no. 12 , 1979 pages 1534 - 1538 PITT, C.G. ET AL 'SUSTAINED DRUG DELIVERY SYSTEMS II: FACTORS AFFECTING RELEASE RATES FROM POLY(E-CAPROLACTONE) AND RELATED BIODEGRADABLE POLYESTERS' cité dans la demande
- CONTRACEPTION vol. 18, no. 4 , Octobre 1978 pages 367 - 394 NASH, H.A. ET AL 'STEROID RELEASE FROM SILASTIC CAPSULES AND RODS' cité dans la demande

## Description

La présente invention se rapporte à de nouvelles compositions à effet progestatif et à leurs procédés d'obtention.

Elle a plus particulièrement pour objet de nouvelles compositions destinées à assurer une contraception prolongée.

Elle a spécifiquement pour objet une préparation sous forme d' implant sous-cutané renfermant une quantité efficace d'un dérivé 3,20-dioxo 6-méthyl 17α-OR₁ , 19-nor pregna 4,6-diène de formule générale I: 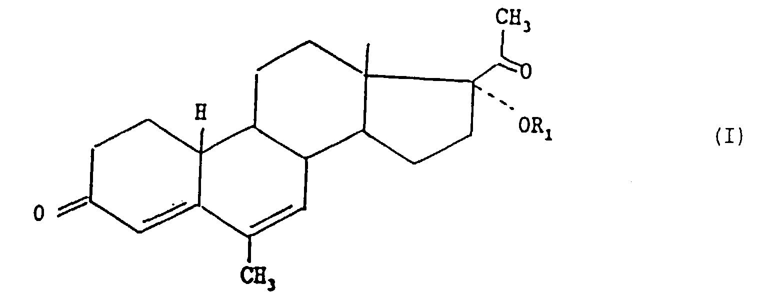 dans laquelle R₁ est de l'hydrogène ou le radical acyle d'un acide organique carboxylique aliphatique, aromatique ou cyclanique ayant de 2 à 16 atomes de carbone en association ou en mélange avec un support et avec un excipient biologiquement acceptable.

Les implants selon l'invention sont faits de matériaux polymériques (hydrophobes, hydrophiles ou biodégradables), utilisés comme vecteurs des composés pharmacologiquement actifs cités précédemment dans la formule I.
Les implants sont, notamment, du type réservoir ou du type matriciel.

Parmi les implants utilisant des matériaux hydrophobes, on peut citer:
- des implants du type réservoir, préparés à partir de polydiméthylsiloxanes comme par exemple Silastic^{(R)}, fabriqués par la société DOW CORNING.
   Ce type d' implant se présente sous la forme d' un tube d' un diamètre extérieur compris entre 2 et 4 mm et d'une épaisseur voisine de 0,4 mm. Les segments de tube sont découpés aux longueurs choisies selon la quantité du dérivé nor-pregnadiénique que l'on désire incorporer. Les extrémités de ces segments de tube sont obturées par une colle de qualité médicale commercialisée également par la société DOW CORNING et qui appartient au groupe de silicone RTV (Room Temperature Vulcanizing). La quantité en dérivé nor-prégnadiénique de formule I incorporé dans le segment de tube s'échelonne de 30 à 80 mg et de préférence de 45 à 65 mg. Cette teneur en principe actif est principalement déterminée par la qualité granulométrique du principe actif (micronisé ou microcristallisé) et de ses caractéristique physico-chimiques (poids moléculaire, solubilité, diffusibilité, coefficient de partage). Le mode d'incorporation du principe actif peut se faire soit à l'état solide, ou en dispersion dans un support biologiquement acceptable et inerte. Le mode de préparation et d'isolement des formes microcristallisées des composés de la présente invention se trouve décrit dans la demande de brevet français 2.668.945 au nom de la demanderesse.
- des implants du type réservoir, préparés à partir de copolymères d' éthylène et d' acétate de vinyle comme par exemple le matériau Elvax ^{(R)} MD 40, fabriqué par la société DUPONT DE NEMOURS.
   Ce type d'implant a déjà été utilisé pour le dispositif intra-utérin Progestasert ^{(R)} Alza, mais au contraire du Progestasert dans le cas des implants selon l'invention, le principe actif est introduit sous forme solide.
- des implants de type matriciel, préparés à partir de polydiméthyl siloxanes.
   Ce type d' implant a déjà été employé chez l'homme (Nash, Robertson et Coll. Contarception, 18 p. 367, (1978). Cet implant du type matrice présente l'inconvénient principalement d'une vitesse élevée de libération du principe actif. Pour pallier cet inconvénient, les implants selon l'invention ont été préparés en introduisant un des dérivés selon la formule I en suspension dans un liquide hydrophile dans lequel le principe actif est insoluble. Cette suspension est mélangée aux polydiméthylsiloxanes, puis la masse totale est réticulée avant de procéder à l'extrusion de l'élastomère sous forme d'un fil cylindrique de dimensions choisies. Ce procédé permet d'obtenir une cinétique de libération d'ordre zéro et non plus fonction de la racine carrée du temps, comme la matrice citée par NASH et Coll.
- des implants de type matriciel, préparés à partir de copolymères d'éthylène et d'acétate de vinyle. Dans ce cas, l'implant est préparé par coulée. En raison de sa nature monolithique, cet implant libère le principe actif selon une cinétique du type racine carrée du temps. L'enrobage partiel par une membrane servant à contrôler la libération de la matrice peut être réalisé, soit avec un polymère d'EVA à faible concentration de vinyl acétate, soit par introduction de la matrice dans un tube de polydiméthylsiloxane (type SILASTIC), pour obtenir des courbes de libération d'ordre zéro.

Parmi les implants utilisant des matériaux hydrophiles, on peut citer :
- des implants de type matriciel, préparés à partir de polyméthacrylates d'hydroxyéthyle comme par exemple Hydron^{(R)}, fabriqué par la société HYDRON MED SCIENCES.
   Ce type d'implant est préparé par polymérisation dans l'alcool suivie de réticulation à l'aide de diméthacrylate d'éthylène glycol. Après désolvatation dans un moule, on obtient à l'état sec (vitreux) un implant dont la cinétique est proche de l'ordre zéro.
   Le procédé de fabrication est inspiré de celui décrit par CHIEN Y.W avec le Norgestomet (J. Pharm. Sci, 1976, 65:488).

Parmi les implants utilisant des matériaux biodégradables, on peut citer :
- des implants de type réservoir, préparés à partir de polycaprolactones, fabriqués selon le procédé décrit par PITT et Coll. (J. Pharm. Sci., 1979, 68 : 1534). Ces implants cylindriques obtenus par extrusion contiennent entre 30 et 50 mg d'un des dérivés nor-pregnadiéniques de formule I, en suspension dans un solvant inerte. Ils ont un diamètre de 2,4 mm.
- des implants de type matriciel, préparés à partir d' un système acide polylactique/acide polyglycolique 50/50, de poids moléculaire de l'ordre de 180.000.
   Ce type d'implant contient 20 à 40 % d'un des dérivés nor pregnadiéniques de formule I. Il est préparé par dissolution du système biodégradable dans le chlorure de méthylène additionné du principe actif et par évaporation du solvant.

L'implant selon l'invention est mis en place à l'aide d'un trocart, après anesthésie locale, dans le tissu sous-cutané de la région fessière ou de l'avant-bras de la femme.

A la fin du traitement, ou à tout moment, l'implant peut être retiré grâce à une petite intervention chirurgicale sous légère anesthésie locale.

Parmi les composés pharmacologiquement actifs qu'il est possible d' insérer dans les implants selon l'invention, on pourra citer :
- le 3,20-dioxo 6-méthyl 17α-hydroxy 19-nor pregna 4,6-diène
- le 3,20-dioxo 6-méthyl 17α-acétoxy 19-nor pregna 4, 6-diène

Les excipients biologiquement compatibles sont des poudres ou des véhicules inertes pharmacologiquement, dégradés progressivement par le milieu biologique et qui servent à diluer, à répartir ou à diviser le principe actif. Des exemples d'excipient ou véhicule sont le lactose, le mannitol, l'urée, l'huile d'olive ou l'huile d'arachide.

Depuis une vingtaine d'années, un grand nombre d'études ont été menées un peu partout dans le monde pour déterminer un mode de contraception efficace et de longue durée. Les essais effectués avec des suspensions injectables massives de progestatif (Provera^{(R)}, etc...) dont la durée de protection s'étend sur environ 6 mois, s'accompagnent souvent d'effets secondaires importants. C'est la raison pour laquelle les expérimentateurs en sont venus à des implants en polymères biocompatibles qui sont insérés soit dans la cavité utérine (Progestasert ^{(R)}), soit dans le tissu sous-cutané (implants).

C'est ainsi que l'on a déjà expérimenté des implants renfermant de nombreux principes actifs stéroïdiens à activité progestative comme la norgestriènone, la noréthindrone, le levonorgestrel.

Cependant, avec ces produits, il est nécessaire, pour obtenir une durée particulièrement longue, d' incorporer une quantité importante de principe actif dans l'implant ou bien d'insérer plusieurs implants.

C'est ainsi que le Norplant^{(R)} qui contient comme principe actif le levonorgestrel, a une durée d'action moyenne de 5 ans pour un ensemble de 6 implants de Silastic^{(R)}. Chaque implant est rempli de 36 mg de levonorgestrel cristallisé, ce qui fait une charge globale de 216 mg en principe actif.

Au contraire, les compositions selon l'invention requièrent l'insertion d'un seul implant mesurant 30 à 45 mm selon le- type d'implant choisi Ce mode d'implantation est considérablement simplifié. Il est plus facilement accepté. L'utilisation comme principe actif d'un dérivé nor-pregnadiènique, répondant à la formule générale I définie précédemment, permet une inhibition de la conception efficace, grévée d'un minimum d'effets secondaires et cette utilisation assure le maintien de saignements périodiques assimilables à des règles. Plus précisement, plusieurs études cliniques ont montré qu'un implant de type réservoir, à base de Silastic^{(R)}, comme décrit précédemment, renfermant comme principe actif de l'acétate de nomégestrol, exerce une activité contraceptive pendant une période d'au moins 12 mois. Une période de 12 mois permet, en outre, une meilleure surveillance des sujets.

Les exemples suivants illustrent l'invention sans toufefois la limiter :

### EXEMPLE I

### PREPARATION DES IMPLANTS TYPE RESERVOIR A BASE DE SILASTIC^{(R)} ET RESULTATS CLINIQUES

Le matériau utilisé est un tube de diméthylpolysiloxane fabriqué par la Société DOW CORNING type 602-265 - référence catalogue.

Des segments de tube sont découpés aux dimensions choisies. Une extrémité du segment est scellée par un matériau adhésif comme le Silastic^{(R)} type A ou type RTV, de qualité médicale.

Les segments sont remplis avec la quantité choisie d'acétate de nomégestrol de qualité granulométrique contrôlée. Les segments sont enfin fermés par le matériau adhésif. Les implants ainsi réalisés sont stérilisés.

Le choix d'une taille particulière pour cet implant de Silastic^{(R)} a été réalisé à la suite d'études préliminaires effectuées sur des volontaires, avec des implants selon l'exemple I de différentes tailles : 20, 30 et 40 mm.
- avec 20 mm, il y a eu deux grossesses
- avec 30 mm, des grossesses sont aussi survenues
- avec 40 mm, il n'y a pas eu de conception mais la proportion d'aménorrhée était élevée.

Le choix a donc porté sur un implant de 35 mm de longueur de remplissage contenant 50 ± 5 mg d'acétate de nomégestrol.

Cet implant a fait l'objet d'une étude réalisée chez 100 femmes, soit 1085 mois-femmes.

Les implants ont été insérés par voie sous-cutanée, après anesthésie locale avec une solution à 2% de procaïne, dans la région fessière. Les sujets étaient des femmes en activité génitale, jeunes (86% avaient moins de trente ans), en période d'activité ovarienne avec des cycles réguliers.

80 sujets participaient encore à l'étude au bout d'un an; 11 sujets ont fait enlever l'implant entre six et onze mois et seulement 9 sujets avant six mois.

Les causes d'abandon ont été :
- 9 cas : préférence pour une autre méthode
- 3 cas : survennue d'irrégularités menstruelles
- 2 cas : prise de poids
- 3 cas : désir d'une grossesse
- 3 cas : effets secondaires (vertiges, dysménorrhée, céphalée, etc..)

Sur un total de 1085 mois-femmes, une grossesse est survenue, ce qui donne un indice de Pearl de 1,1%.

Des saignements ressemblant à des règles surviennent chez toutes les femmes, mais à un rythme variable d'une femme à l'autre. Parmi les 80 femmes qui ont eu un an d'utilisation, le nombre moyen d'épisodes de saignements est de 12,24 ± 0,29, avec une durée moyenne en jours de 5,24 ± 0,11. Le tableau I rassemble les caractères de cet effet chez les femmes traitées.

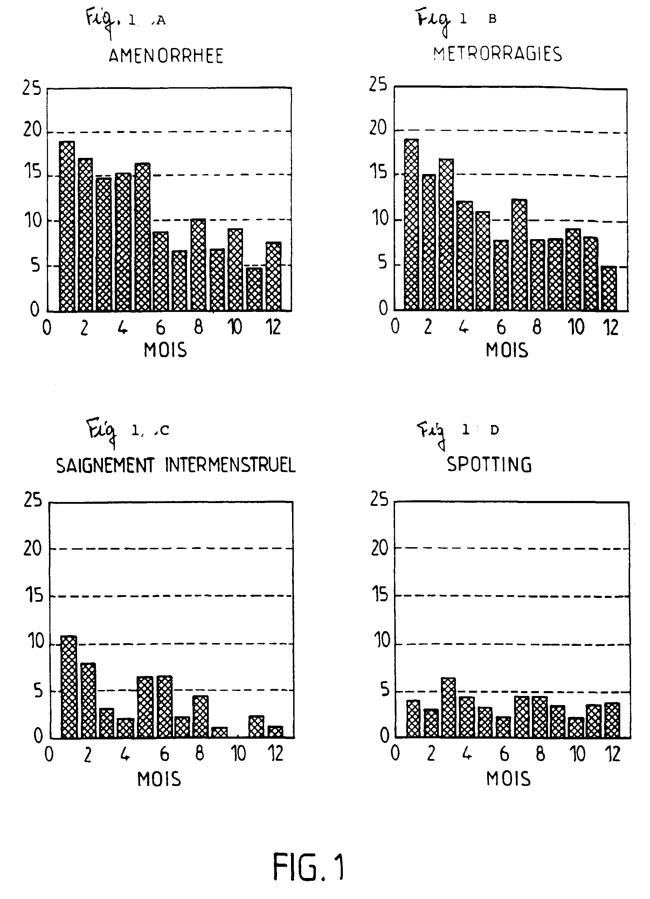

La figure 1 rassemble les anomalies menstruelles (aménorrhée, métrorragies, spottings) notées chez les vingt premières femmes qui ont atteint un an d'utilisation.

Une aménorrhée survient chez 19% des femmes le premier mois, puis l'incidence diminue avec le temps (4,7% au 11ème mois). La fréquence des saignements intermenstruels diminue aussi avec le temps (de 11% le premier cycle, à 1% le dernier mois d'utilisation).

**TABLEAU I**

| **IMPLANT D'ACETATE DE NOMEGESTROL** Schéma menstruel pendant une utilisation de 12 mois (N=80 femmes) | | |
|---|---|---|
| Paramètres | Moyenne | E.S |
| Nombre d'épisodes de saignements (+) | 12,24 | 0,29 |
| Durée moyenne des saignements (jours) | 5,24 | 0,11 |
| Nombre total des jours de saignement | 64,19 | 2,75 |
| Nombre total de jours avec spotting | 7,24 | 1,50 |
| Nombre de jours de saignement et de spotting | 71,42 | 3,22 |
| Durée moyenne entre deux saignements consécutifs (++) | 24,51 | 0,60 |
| Durée moyenne des cycles en jours (+++) | 29,80 | 0,60 |
| Plus long épisode de saignement (jours) | 10,70 | 0,92 |
| Plus long intervalle sans saignement (jours) | 57,86 | 4,13 |

| | | |
|---|---|---|
| (+) un épisode de saignement est représenté par un ou plusieurs jours consécutifs de saignement (à l'exception du spotting) | | |
| (++) un intervalle sans saignement est l'intervalle entre deux épisodes de saignement. | | |
| (+++) un cycle comprend un épisode de saignement et un intervalle sans saignement. | | |

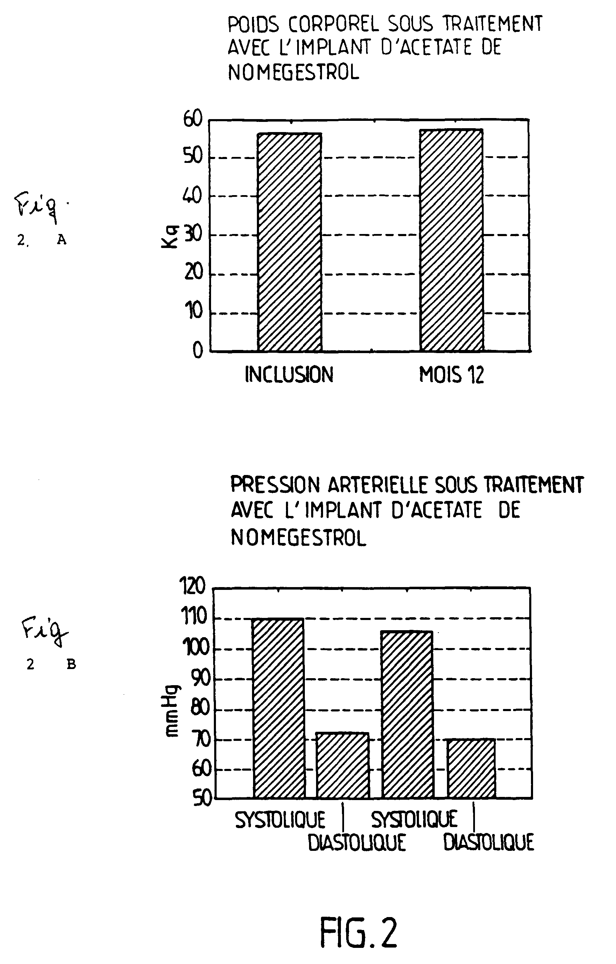

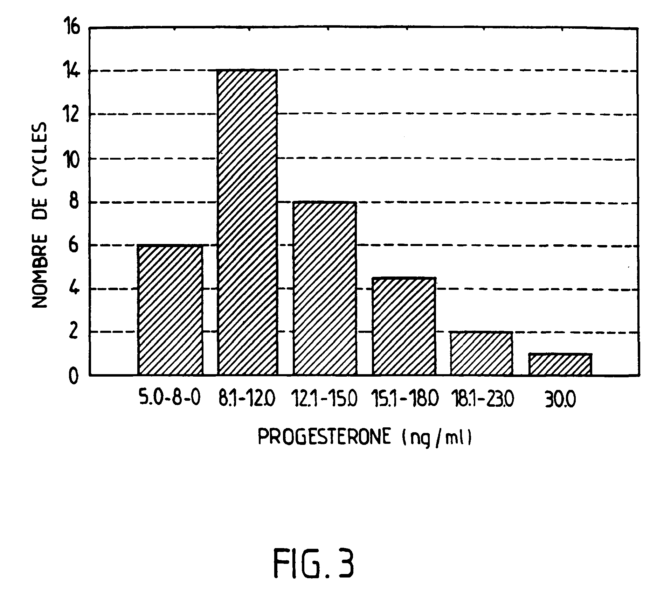

La figure 2 montre les variations du poids (n = 77) et ce pression artérielle (n = 79) chez les femmes ayant porté l'implant pendant un an. Il existe une baisse statistiquement significative de la pression systollique (p= 0,02). Le poids augmente en moyenne de 1,1 kg de façon significative (p = 0,05).

Des dosages d'hormones ont été effectués chez des sujets ayant un implant :
a) Des dosages ponctuels de progestérone en deuxième partie de "cycle", sur douze sujets et soixante cycles ont d'abord été réalisés.
   Dans trente-six cycles, un taux de progestérone supérieur à 3 ng/ml témoigne de la survenue d'une ovulation. Ces valeurs sont indiquées dans le tableau 3.
b) Chez cinq femmes, des prélèvements au moins hebdomadaires, pour des dosages d'estradiol et de progestérone, ont été effectués lors du cycle témoin, pendant les cinq ou six premiers cycles de traitement, puis pendant les onzième et douzième cycles.

Avant l'insertion des implants, les cycles étaient toujours ovulatoires, avec un taux de progestérone de 8,1 ± 4,2 ng/ml et de 10,1 ± 3,5 ng/ml, respectivement au 18ème et 24ème jour du cycle. Sous traitement par l'implant d'acétate de nomégestrol, les taux de progestérone ont été inférieurs, mais la différence par comparaison aux taux d'inclusion n'est devenue significative qu'à partir du 24ème jour du deuxième cycle (p < 0,05).

Dans l'ensemble, les taux les plus bas ont été obtenus au cours des quatre premiers cycles de traitement. Au 12ème cycle de traitement, les valeurs moyennes ont été normales, respectivement de 7,1 ± 1,1 et de 15,6 ± 4,7 ng/ml au 18ème et au 24ème jour.

Sur l'ensemble des trente-huit cycles étudiés, une ovulation (taux de progestérone supérieur ou égal à 5 ng/ml) a été observée dans 24 cas (63,2%). Au cours des premiers cycles de traitement de chacune des cinq patientes, la progestérone a toujours été inférieure à 5 ng/ml. Au cours du 12ème cycle, le taux de progestérone plasmatique était toujours compatible avec la survenue d'une ovulation.

En conclusion, l'implant de 35 mm contenant 48 mg d'acétate de nomégestrol est un contraceptif efficace pendant une période d'au moins 12 mois, sans entrainer d'aménorrhées, dont on sait qu'elles sont difficilement acceptées par les femmes.

La tolérance, tant gynécologique que générale, est bonne.

### EXEMPLE II

### IMPLANT D'ACETATE DE NOMEGESTROL DU TYPE MATRICIEL

On prépare une masse générale, formatrice de l'élastomère à partir de 70 à 75% d'un dérivé polyorganosiloxane à groupements silanol terminaux et de 24 à 29% de silice, contenant entre 0,2% et 0,5% d'un catalyseur choisi.

On prépare, d'autre part, un mélange contenant 50% d'un des principes actifs répondant à la formule I, II ou III, en suspension dans un solvant hydrophile, le tout mélangé intimement dans 50% d'un polydiorganosiloxane.

Le mélange contenant le principe actif a été incorporé dans la composition formatrice de l'élastomère suivant les méthodes classiques connues dans la technique.

On charge le mélange total dans une extrudeuse de façon à obtenir un extrudat continu, de diamètre compris entre 2 et 3 mm selon le type d'implant.

Cet extrudat est réticulé par passage en étuve ventilée à une température comprise entre 30 et 80°C.

Cet extrudat est ensuite coupé aux dimensions choisies de raçon a obtenir des implants de forme tubulaire contenant 30 à 60 mg de principe actif.

Pour les essais "in vitro", on a utilisé des implants d'un poids voisin de 120 mg contenant 25% de principe actif.

### EXEMPLE III

### ETUDE DE LIBERATION "IN VITRO" DE L'ACETATE DE NOMEGESTROL (3,20-dioxo 6-méthyl 17α-acétoxy 19-nor pregna 4,6-diène) DANS DIFFERENTS IMPLANTS

On a procédé à une comparaison des vitesses de libération du principe actif incorporé dans trois implants de type matriciel et dans un implant de type réservoir.

Les implants ont été placés dans des récipients contenant une quantité connue d'eau préalablement filtrée et désaérée. Ce milieu est changé toutes les 24 heures et l'essai a duré 14 jours.

L'acétate de nomégestrol libéré dans le milieu a été dosé par spectrophotométrie UV à 296 nm. Pour chaque formulation, le dosage a été répété trois fois. Les résultats sont exprimés sous forme de moyenne de trois dosages et en µg de principe actif libéré par 24 heures. Ces résultats sont rassemblés dans les tableaux II et III.

Deux des trois implants de type matriciel ont présenté un profil de libération très semblable, avec une libération de 110 µg/jour à l'état d'équilibre. Au bout de 14 jours, environ 14% du principe actif étaient libérés.

La libération du principe actif de l'implant de type réservoir a été deux fois plus lente; dans tous les cas cependant la cinétique était parallèle.

Le troisième implant, de type matriciel, a donné un profil de libération sensiblement différent avec une libération immédiate plus importante.

### EXEMPLE IV

### PREPARATION DES IMPLANTS DU TYPE MATRICIEL A BASE DE COPOLYMERE D'ETHYLENE ACETATE DE VINYLE (EVA)- RESULTATS DE L' ETUDE DE LIBERATION "IN VITRO" DE L'ACETATE DE NOMEGESTROL

Les implants sont préparés par broyage et lavage de l'EVA, puis séchage et mélange avec l'acétate de Nomegestrol dans des proportions de 165 mg d'EVA pour 55 mg de principe actif (soit 3 parties d'EVA pour 1 partie d'acétate de Nomegestrol).

Le mélange une fois homogène est coulé, extrudé et comprimé à une température comprise entre 50°C et 65°C, pour obtenir un extrudat cylindrique de 2,3 mm de diamètre.

Celui-ci est coupé tous les 40 mm pour obtenir un implant de la longueur désirée. Celui-ci est d'un poids voisin de 220 mg ± 10 mg et contient 55 mg d'acétate de Nomegestrol ± 5mg.

Cette matrice est prête à être recouverte d'une membrane poreuse pour réguler la vitesse de libération (PDMS ou EVA).

### ETUDE DE LIBERATION DE LA MATRICE

Chaque implant est placé dans un récipient contenant une quantité d'eau déionisée, de 20 ml (pour 24 heures).

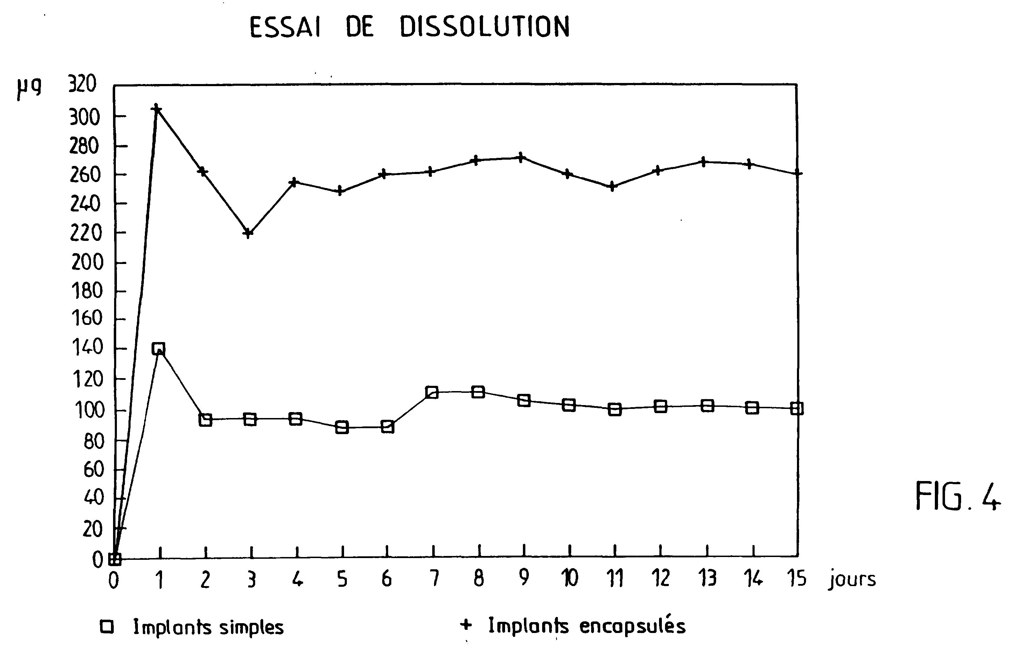

Ce milieu est remplacé toutes les 24 heures et l'expérience dure, selon les essais, de 15 à 31 jours. La teneur en acétate de Nomegestrol dans le milieu est dosé par spectrophométrie UV à 296 nm. Les résultats sont exprimés en µg de principe actif libéré par 24 heures. Ces résultats sont rassemblés sur la figure 4 comparativement à un implant en copolymère d'EVA. Ils montrent la régularité de la libération de principe actif après encapsulation par une membrane poreuse et en même temps, la moindre libération de l'acétate de Nomegestrol en fonction de la durée.

### EXEMPLE V

### PREPARATION DES IMPLANTS TYPE MATRICIEL A BASE DE COPOLYMERE ACETATE DE VINYLE/ETHYLENE MIS EN TUBING DE POLYDIMETHYLSILOXANE

La matrice est préparée selon l'exemple II et introduite dans un tube de polydiméthylsiloxane Medical grade (SILASTIC), fermé aux extrêmités par de la colle SILASTIC Medical Adhesive Silicone Type A stérile.

L'implant terminé a un diamètre de 3 mm et est constitué d'une matrice cylindrique de copolymère acétate de vinyle/éthylène de #2,3 mm de diamètre et de 40 mm de longueur.

La longueur totale est de 42 mm après obturation par la colle des deux extrémités.

### ETUDE DE LIBERATION

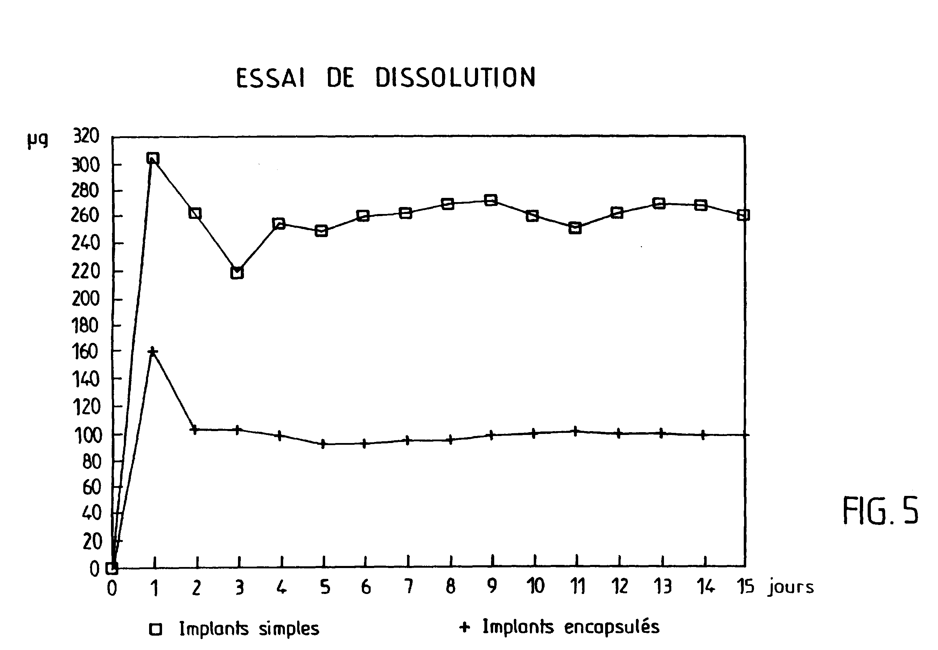

On procède selon le mode opératoire et la méthode de dosage qu'à l'exemple III.
Les résultats sont exprimés en µg d'acétate de Nomégestrol libéré par 24 heures. Ces résultats sont représentés sur la figure 5 en comparaison avec ceux obtenus dans une matrice d'EVA seule.

**TABLEAU II**

| **LIBERATION D'ACETATE DE NOMEGESTROL (en µg/jour) A PARTIR D'IMPLANTS MATRICIELS (F 7571-113-11 : F 7571-113-13 : F 7571-113-15) ET DE TYPE RESERVOIR (Implant THERAMEX) selon l'invention** | | | | |
|---|---|---|---|---|
| JOUR | F7571-113-11 | F7571-113-13 | F7571-113-15 | IMPLANT THERAMEX |
| 1 | 184.4 | 210.2 | 186.0 | 103.4 |
| 2 | 142.9 | 206.7 | 146.1 | 71.4 |
| 3 | 128.4 | 209.2 | 132.0 | 65.5 |
| 7 | 492.6 | 663.7 | 490.0 | 201.5 |
| 8 | 111.9 | 189.2 | 116.5 | 54.4 |
| 9 | 113.7 | 187.5 | 114.2 | 55.5 |
| 10 | 113.0 | 181.8 | 113.9 | 54.5 |
| 13 | 325.8 | 444.0 | 344.4 | 143.0 |
| 14 | 107.9 | 130.5 | 109.4 | 50.1 |

**TABLEAU III**

| **LIBERATION CUMULEE (en µg) D'ACETATE DE NOMEGESTROL A PARTIR D' IMPLANTS MATRICIELS (F 7571-113-11 : F 7571-113-13 : F 7571-113-15) ET DE TYPE RESERVOIR (Implant THERAMEX) selon l'invention** | | | | |
|---|---|---|---|---|
| JOUR | F7571-113-11 | F7571-113-13 | F7571-113-15 | IMPLANT THERAMEX |
| 1 | 184.4 | 210.2 | 186.0 | 103.4 |
| 2 | 327.3 | 416.9 | 332.1 | 174.8 |
| 3 | 455.7 | 626.1 | 464.1 | 240.3 |
| 7 | 948.3 | 1289.8 | 954.1 | 441.8 |
| 8 | 1060.2 | 1479.0 | 1070.6 | 496.2 |
| 9 | 1173.9 | 1666.5 | 1184.8 | 551.7 |
| 10 | 1286.9 | 1848.3 | 1298.7 | 606.2 |
| 13 | 1612.7 | 2292.3 | 1643.1 | 749.2 |
| 14 | 1720.6 | 2422.8 | 1752.5 | 799.3 |

## Revendications

1. De nouvelles compositions sous forme d'implants sous-cutanés caractérisées en ce qu'elles renferment une quantité efficace d' un dérivé 3,20-dioxo 6-méthyl 17α-OR₁ 19-nor pregna 4,6-diène de formule I 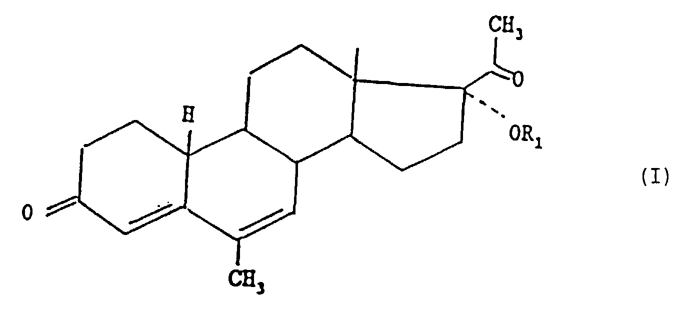 dans laquelle R₁ est de l'hydrogène ou le reste acyle d'un acide organique carboxylique aliphatique, aromatique ou cyclanique ayant de 2 à 16 atomes de carbone,
en association ou en mélange avec un excipient physiologiquement acceptable dans un support compatible physiologiquement.

2. Une composition selon la revendication 1° dans laquelle le principe actif est sous forme micronisée, microcristalline, broyée ou amorphe.

3. Une composition selon la revendication 1 et 2° dans laquelle le principe actif est d'une qualité telle que celle obtenue selon le procédé de microcristallisation.

4. Une composition selon les revendications 1 et 2° dans laquelle le matériau support est un polymère biocompatible et stérilisable choisi dans le groupe formé des polydiméthylsiloxanes, des copolymères d'éthylène et d'acétate de vinyle, des polyméthacrylates d'hydroxyéthyle, des polycaprolactones et des systèmes acide polylactique/acide polyglycolique.

5. Une composition selon l'une des revendications 1 à 4° dans laquelle le principe actif est le 3,20-dioxo 6-méthyl 17α-OR₁ 19-nor pregna 4,6-diènes dans lequel R₁ est le radical acyle d'un acide organique aliphatique carboxylique, ou de l'hydrogène.

6. Une composition selon la revendication 5° dans laquelle le principe actif est un 3, 20-dioxo 6-méthyl 17α-hydroxy 19-nor pregna 4,6-diène.

7. Une composition selon la revendication 5° dans laquelle le principe actif est le 3,20-dioxo 6-méthyl 17α-acétoxy 19-nor pregna 4,6-diène.

8. Une composition sous forme d'implant sous-cutané selon l'une des revendications 1 à 7° dans laquelle le matériau polymérique est le diméthyl polysiloxane.

9. Une composition selon l'une des revendications 1 à 8° dans laquelle la quantité efficace de dérivé 6-méthyl 17α- OR₁ 20-oxo 19-nor pregna 4,6-diènique s'échelonne de 30 à 80 mg par implant sous-cutané.

10. Une composition selon la revendication 9° dans laquelle la teneur 6-méthyl 17α- OR₁ 20-oxo 19-nor pregna 4,6-diène s'échelonne de 35 à 65 mg par implant sous-cutané.

11. Une composition selon la revendication 9° et la revendication 10° dans laquelle la teneur en 3,20-dioxo 6-méthyl 17α-acétoxy 19-nor pregna 4,6-diène s'échelonne de 35 à 65 mg par implant sous-cutané.

12. Une composition selon la revendication 11° dans laquelle le dérivé 6-méthyl 17α-acétoxy 20-oxo 19-nor pregna 4,6-diènique est d'une qualité particulière comme celle obtenue par le procédé de microcristallisation.

13. Une composition selon l'une des revendications 1 à 12° dans laquelle l'implant est formé d'un segment de matériau polymérique de forme cylindrique, dont la longueur est comprise entre 20 à 50 mm et le diamètre 2 à 4 mm.

14. Une composition selon l'une des revendications 1 à 13° dans laquelle l'implant est formé d'un segment de matériau polymérique à base de copolymère éthylène/acétate de vinyle (EVA), dont la longueur est comprise entre 20 à 50 mm et le diamètre varie de 2 à 4 mm.

15. Une composition selon la revendication 14° qui renferme de 50 à 80 mg de principe actif selon la revendication 1°.

16. Une composition selon la revendication 14° dans laquelle l'implant du type matriciel, à base de copolymère éthylène/acétate de vinyle, est recouvert d'une membrane à base d'éthylène/acétate de vinyle à faible concentration d'acétate de vinyle.

17. Une composition selon l'une des revendications 14 à 16° dans laquelle l'implant en copolymère éthylène/acétate de vinyle renfermant le principe actif, est introduit dans un tube de poly (diméthylsiloxane), et que l'on ferme aux deux extrémités par une colle siliconée l'ensemble ainsi formé.

18. Un procédé de réalisation des implants selon les revendications 1 à 13° qui consiste en ce que l'on coupe un tube de matériau polymérique creux comportant un canal intérieur, à une longueur comprise entre 20 et 50 mm, en ce que l'on remplit le canal intérieur avec une quantité efficace d'un dérivé selon la revendication 1° ou la revendication 2° puis on scelle les extrémités du tube avec une colle spécifique et on stérilise le tube par des moyens physiques.

## Claims

1. Novel compositions in the form of subcutaneous implants characterized in that they contain an efficient amount of a 3,20-dioxo 6-methyl 17α-OR₁ 19-nor pregna-4,6 diene derivative of formula 1 : 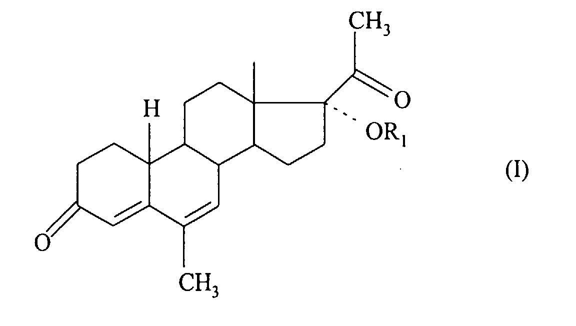 wherein R₁ is hydrogen, or the acyl residue of an aliphatic, aromatic or cyclanic organic carboxylic acid having from 2 to 16 carbon atoms
in admixture or conjunction with a physiologically acceptable diluent in a physiologically compatible carrier.

2. A composition according to claim 1 wherein the active ingredient is under micronized, micro cristalline, ground or amorphous form.

3. A composition according to claim 1 and 2, wherein the active ingredient is of such as quality than that produced according to the microcristallization process.

4. A composition according to claims 1 and 2, wherein the carrier material is a biocompatible and sterilizable polymer selected from the group consisting of polydimethyl siloxanes, ethylene / vinyl acetate copolymers, hydroxyethyl polymethacrylates, polycaprolactons and polylactic acid / polyglycolic acid systems.

5. A composition according to any of the claims 1 to 4 wherein the active ingredient is a 3,20-dioxo 6-methyl 17α-OR₁ 19-nor pregna 4,6 - dien wherein R₁ is the acyl residue of an organic aliphatic carboxylic acid or a hydrogen.

6. A composition according to claim 5 wherein the active ingredient is 3,20-dioxo 17α-hydroxy 6-methyl 19-nor pregna 4,6-diene.

7. A composition according to claim 5 wherein the active ingredient is 3,20-dioxo 17α-acetoxy 6-methyl 19-nor pregna 4,6-diene.

8. A composition in the form of subcutaneous implant according to any of the claims 1 to 7 wherein the polymeric material is dimethyl polysiloxane.

9. A composition according to any of claims 1 to 8 wherein the efficient amount of 6-methyl 17α-OR₁ 20-oxo 19-nor pregna 4,6 diene ranges from 30 to 80 mg per subcutaneous implant.

10. A composition according to claim 9 wherein the content in 6-methyl 17α-OR₁ 20-oxo 19-nor pregna 4,6-diene ranges from 35 to 65 mg per subcutaneous implant.

11. A composition according to claims 9 and 10, wherein the content in 3,20-dioxo 6-methyl 17α-acetoxy 19-nor pregna 4,6-diene ranges from 35 to 65 mg per subcutaneous implant.

12. A composition according to claim 11 wherein the 6-methyl 17α-acetoxy 20-oxo 19-nor pregna 4,6-dien derivative, is of a specific quality such as that obtained through the microcristallization process.

13. A composition according to one of the claims 1 to 12 wherein the implant is made of a segment of a polymeric material, the length of which, ranges from 20 to 50 mm and the diameter of which from 2 to 4 mm.

14. A composition according to one of the claims 1 to 13 wherein the implant is made of a segment of a polymeric material based on a ethylene/vinyl acetate (EVA) the length of which ranges from 20 to 50 mm and the diameter from 2 to 4 mm.

15. A composition according to claim 14 which contain from 50 to 80 mg of active ingredient according to claim 1.

16. A composition according to claim 14 wherein the implant of the matrix type based on ethylene/vinyl acetate copolymer, is covered with a membran based on ethylene/vinyl acetate with a low content of vinyl acetate.

17. A composition according to one of the claims 14 to 16 wherein the implant in ethylene/vinyl acetate copolymer, containing the active ingredient, is introduced in a tubing of poly(dimethyl siloxane) and wherein the thus-formed ensemble is closed at both ends by means of a siliconated adhesive.

18. A process for producing the implants according to claims 1 to 13 which consists in that a tube of holow polymeric material, having an internal channel is cut at a length comprised between 20 and 50 mm, in that the internal channel is filled with an efficient amount of derivative according to claim 1 or 2, then the ends of the tube are sealed with a specific glue and the tube is sterilized using physical means.

## Patentansprüche

1. Neue Zusammensetzungen in Form von subkutanen Implantaten, dadurch gekennzeichnet, daß sie eine wirksame Menge eines 3,20-Dioxo-6-methyl-17α-OR₁-19-norpregna-4,6-dien-Derivats mit der Formel I 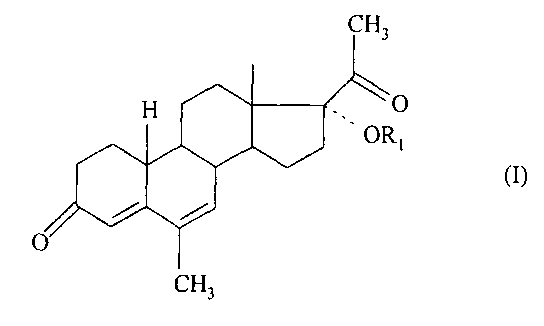 einschließen, wobei R₁ Wasserstoff oder der Acylrest einer aliphatischen, aromatischen oder naphthenischen organischen Carbonsäure mit 2 bis 16 Kohlenstoffatomen ist,
in Kombination oder Abmischung mit einem physiologisch akzeptablen Exzipienten in einem physiologisch verträglichen Träger.

2. Zusammensetzung nach Anspruch 1, wobei der Wirkstoff in mikronisierter, mikrokristalliner, gemahlener oder amorpher Form vorliegt.

3. Zusammensetzung nach Anspruch 1 und 2, wobei der Wirkstoff von einer Qualität ist, wie sie nach dem Mikrokristallisationsverfahren erzielt wird.

4. Zusammensetzung nach Anspruch 1 und 2, wobei das Trägermaterial ein biologisch verträgliches und sterilisierbares Polymer ist, das aus der Gruppe gewählt wird, die aus Polydimethylsiloxanen, Ethylen/Vinylacetat-Copolymeren, Hydroxyethyl-Polymethylacrylaten, Polycaprolactonen und Polymilchsäure/Polyglycolsäure-Systemen gebildet wird.

5. Zusammensetzung nach einem der Ansprüche 1 bis 4, wobei der Wirkstoff 3,20-Dioxo-6-methyl-17α-OR₁-19-norpregna-4,6-dien ist, bei dem R₁ das Acylradikal einer aliphatischen organischen Carboxylsäure oder Wasserstoff ist.

6. Zusammensetzung nach Anspruch 5, wobei der Wirkstoff ein 3,20-Dioxo-6-methyl-17α-hydroxy-19-norpregna-4,6-dien ist.

7. Zusammensetzung nach Anspruch 5, wobei der Wirkstoff 3,20-Dioxo-6-methyl-17α-acetoxy-19-norpregna-4,6-dien ist.

8. Zusammensetzung in Form eines subkutanen Implantats nach einem der Ansprüche 1 bis 7, wobei das Polymermaterial Dimethylpolysiloxan ist.

9. Zusammensetzung nach einem der Ansprüche 1 bis 8, wobei die Wirksame Menge des 6-Methyl-17α- OR₁-20-oxo-19-norpregna-4,6-dien-Derivats von 30 bis 80 mg pro subkutanem Implantat gestaffelt ist.

10. Zusammensetzung nach Anspruch 9, wobei der Gehalt an 6-Methyl-17α- OR₁-20-oxo-19-norpregna-4,6-dien von 35 bis 65 mg pro subkutanem Implantat gestaffelt ist.

11. Zusammensetzung nach Anspruch 9 und 10, wobei der Gehalt an 3,20-Dioxo-6-methyl-17α-acetoxy-19-norpregna-4,6-dien von 35 bis 65 mg pro subkutanem Implantat gestaffelt ist.

12. Zusammensetzung nach Anspruch 11, wobei das 6-Methyl-17α-acetoxy-20-oxo-19-norpregna-4,6-dien-Derivat von einer besonderen Qualität ist, wie sie durch das Mikrokristallisationsverfahren erzielt wird.

13. Zusammensetzung nach einem der Ansprüche 1 bis 12, wobei das Implantat durch ein Segment eines Polymermaterials von zylindrischer Form gebildet wird, dessen Länge zwischen 20 und 50 mm und dessen Durchmesser zwischen 2 und 4 mm beträgt.

14. Zusammensetzung nach einem der Ansprüche 1 bis 13, wobei das Implantat durch ein Segment eines Polymermaterials auf der Basis eines Ethylen/Vinylacetat-Copolymers (EVA) gebildet ist, dessen Länge zwischen 20 und 50 mm beträgt und dessen Durchmesser zwischen 2 und 4 mm schwankt.

15. Zusammensetzung nach Anspruch 14, die zwischen 50 und 80 mg Wirkstoff nach Anspruch 1 enthält.

16. Zusammensetzung nach Anspruch 14, wobei das Implantat vom Matrixtyp auf der Basis eines Ethylen/Vinylacetat-Copolymers mit einer Membran auf Ethylen/Vinylacetat-Basis mit geringer Vinylacetatkonzentration bedeckt ist.

17. Zusammensetzung nach einem der Ansprüche 14 bis 16, wobei das Implantat aus Ethylen/Vinylacetat-Copolymer, das den Wirkstoff einschließt, in ein Rohr aus Poly(dimethylsiloxan) eingeführt wird und daß man das auf diese Weise gebildete Ganze an den beiden Enden durch einen Silikonkleber verschließt.

18. Verfahren zur Herstellung von Implantaten nach den Ansprüchen 1 bis 13, darin bestehend, daß man ein hohles Rohr aus Polymermaterial, das einen inneren Kanal aufweist, auf eine Länge zwischen 20 und 50 mm zuschneidet, und daß man den inneren Kanal mit einer wirksamen Menge eines Derivats nach Anspruch 1 oder 2 füllt und dann die Enden des Rohrs mit einem spezifischen Kleber versiegelt und das Rohr durch physikalische Mittel sterilisiert.
